Europäisches Patentamt

European Patent Office

Office européen des brevets

(19)

(11) Publication number: 0 471 293 A2

## EUROPEAN PATENT APPLICATION

(12)

(21) Application number: 91113327.0

(22) Date of filing: 08.08.91

(51) Int. Cl.⁵: G01N 33/50, //G01N33/68, C07K7/64

(30) Priority: 15.08.90 US 567840

(43) Date of publication of application:
19.02.92 Bulletin 92/08

(84) Designated Contracting States:
AT BE CH DE DK ES FR GB GR IT LI NL SE

(71) Applicant: ABBOTT LABORATORIES
CHAD-0377, AP6D/2, One Abbott Park Road
Abbott Park, Illinois 60064-3500(US)

(72) Inventor: Meucci, Victoria P.
1660 N. LaSalle, Apt.311
Chicago, Illinois(US)
Inventor: Zajac, Mariola B.
1660 N. Halstead
Chicago, Illinois(US)

(74) Representative: Modiano, Guido et al
MODIANO, JOSIF, PISANTY & STAUB
Modiano & Associati Via Meravigli, 16
I-20123 Milano(IT)

(54) Solubilization reagent for biological test samples.

(57) A solubilization reagent for use in analytical systems for the determination of hydrophobic analytes in a biological test sample, particularly analytical systems employing specific binding proteins for such analytes, such as in fluorescent polarization immunoassays, is disclosed. The solubilization reagent dissociates analytes from various components of a biological test sample, such as cellular material, phospholipids, proteins and the like, at substantially low concentrations of such solubilization reagent while, at the same, minimizing the denaturation of specific binding proteins, such as, for example, antibodies, which may be present in an analytical system. Preferably, such surfactant is alkyl-oxy-(polyethylene-oxy-propylene-oxy-sopropanol) or N-tetradecyl-n,n-demethyl-3-ammonio-1-propane sulfonate, and may further comprise saponin.

FIG. 1

## Field of the Invention

The present invention relates to reagents which are useful for extracting analytes from a liquid test sample. In particular, the present invention relates to reagents which facilitate the dissociation of analytes, particularly hydrophobic analytes, from the components of a biological test sample to permit the measurement of such analytes present therein.

## Background of the Invention

The monitoring of therapeutic drug levels and other analytes in biological fluids such as serum, plasma, whole blood, urine and the like has become very useful to provide physicians with information to aid in proper patient management. For example, adjustment of patient dosage, achievement of optimal therapeutic effects, and avoiding useless subtherapeutic or harmful toxic dosage levels can be provided. Conventional techniques which are employed to monitor drug levels or detect other analytes are known and include radioimmunoassays and nonisotopic assays such as fluorescence polarization immunoassays. However, such techniques produce inconsistencies in results when determining the amount or presence of hydrophobic analytes because of their intracellular relationship with various cellular components of a biological test sample. Accordingly, when such analytes remain associated with such cellular components, the detection of such analytes in an analytical system is difficult, and in some instances impossible, particularly when such analytes are present at particularly low levels.

Although various reagents have been described to extract various analytes for analysis, such as Triton X-100[R], Tweens[R], sodium dodecyl sulfate and saponin, the use of such reagents suffer from a number of disadvantages, particularly where such analysis involves reagents such as specific binding proteins, antibodies, and the like. For example, such reagents, in many cases, do not achieve complete cell lysis wherein in the case of hydrophobic analytes, a significant amount of such analytes could remain associated with cellular components and thereby not made available for analysis. Similarly, the presence of such reagents in, for example, an immunoassay system, will result in significant denaturation of specific binding proteins or antibodies employed in such immunoassays to thereby reduce the binding activity of such proteins and antibodies. Moreover, the use of such reagents to dissociate analytes from various cellular components and other materials which may be present in a liquid test sample can have a dramatic effect on the integrity of reagents employed in various analytical systems, particularly where such reagent are employed at high concentrations in order to achieve such dissociation.

## Summary of the Invention

The present invention relates to the discovery that analytical systems for the determination of hydrophobic analytes in a biological test sample, particularly analytical systems employing specific binding proteins for such analytes, can be substantially improved by employing the solubilization reagent of the present invention which serves to dissociate analytes from various components of a biological test sample, such as cellular material, phospholipids, proteins and the like. In particular, such solubilization reagent has unexpectedly and surprisingly been found to dissociate hydrophobic analytes from such components, particularly cellular components, at substantially low concentrations of such solubilization reagent while, at the same, minimize the denaturation of specific binding proteins, such as, for example, antibodies, which may be present in an analytical system. The solubilization reagent of the present invention is particularly useful in a fluorescent polarization immunoassay for the determination of hydrophobic analytes such as cyclosporine and the like.

The solubilization reagent of the present invention comprises from between about 1.5% (w/v) and about 10% (w/v), preferably about 2% (w/v), of a surfactant having either nonionic characteristics or zwitterionic characteristics wherein such surfactant is capable of dissociating substantially all of a hydrophobic analyte from the components of a biological test sample. Preferably, such surfactant is either a nonionic polyglycol detergent, such as alkyl-oxy(polyethylene-oxy-propylene-oxy-isopropanol), or a zwitterionic detergent, such as N-tetradecyl-N,N-demethyl-3-ammonio-1-propanesulfonate. The solubilization reagent may further comprise from between about 0% (w/v) and about 25% (w/v) of saponin.

## Brief Description of the Drawings

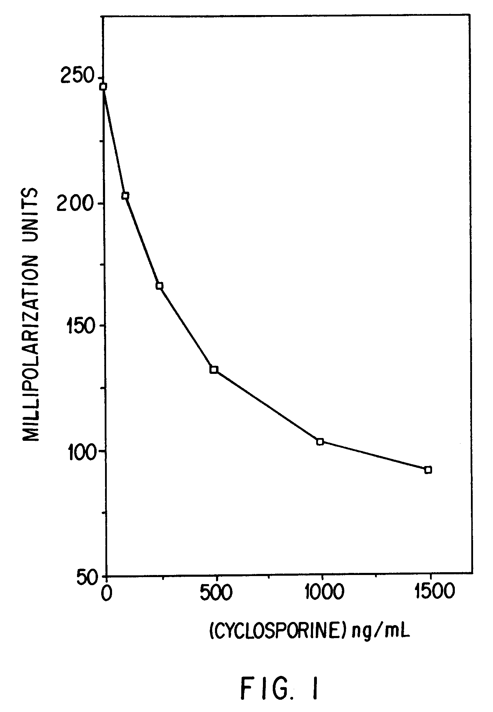

Fig. 1 illustrates a calibration curve employed to determine the amount cyclosporine from a whole blood sample in a fluorescent polarization immunoassay employing the solubilization reagent of the present invention.

## Detailed Description of the Invention

The solubilization reagent of the present invention dissociates analytes, particularly hydrophobic analytes, from a biological test sample such as whole blood, serum, plasma, urine, spinal fluid, and the like. As contemplated by the present invention, hydrophobic analytes include, but are not intended to be limited to, steroids, drugs such as cyclosporine, and the like.

In particular, the solubilization reagent dissociates such analytes from cellular material, such as erythrocytes, populations of leucocytes, such as lymphpocytes, phospholipids, proteins, and the like, which may be present in a biological test sample, to thereby render such analytes readily available for measurement by a desired analytical system. Although the solubilization reagent is particularly useful in analytical systems for determining hydrophobic analytes employing specific binding proteins, especially immunoassay systems, the solubilization reagent can be be employed in other assay systems as well, such as radioactive assays and the like.

Where it is desireable to employ a non-ionic surfactant in the solubilization reagent according to the present invention, such non-ionic surfactant is preferably a nonionic polyglycol detergent such as alkyloxy(polyethyleneoxypropyleneoxy)-isopropanol and the like, also commonly known as Tergitol[R]. Where it desireable to employ a zwitterionic surfactant in the solubilization reagent according to the present invention, such zwitterionic surfactant is selected from the group consisting of N-tetradecyl-N,N-demethyl-3-ammonio-1-propanesulfonate, N-octyl-N,N-dimethyl-3-ammonio-1-propanesulfonate, N-decyl-N,N-dimethyl-3-ammonio-1-propanesulfonate, N-dodecyl-N,N-dimethyl-3-ammonio-1-propanesulfonate, N-hexadecyl-N,N-dimethyl_3-ammonio-1-propanesulfonate, and the like.

In addition to either a non-ionic surfactant or a zwitterionic surfactant as described above, the solubilization reagent according to the present invention may further comprise a glycoside, such as saponin.

According to the present invention, the solubilization reagent is capable of lysing substantially all of the various cellular components which may be present in a biological test sample, and dissociate substantially all of the desired analyte from other biological test sample components in order to render such analyte available for analysis. In particular, the solubilization reagent according to the present invention is capable of providing substantially complete cell lysis of, for example, cellular populations such as erythrocytes, leukocytes and the like, for recovery of substantially all of the desired analyte contained therein. In addition, the solubilization reagent is also capable of dissociating the desired analyte from other components which may be present in a biological test sample, such as cellular material, phospholipids, proteins, and the like, to which such analyte could nevertheless remain associated with and thereby not made available for analysis.

The solubilization reagent according to the present invention has unexpectedly and surprisingly been found to achieve such cell lysis and dissociation of analyte at substantially low concentration. In particular, the use of the solubilization reagent at a concentration as low as 1.5% (w/v) of the non-ionic or zwitterionic surfactant, with saponin, has been found to be effective for such cell lysis and dissociation of the analyte. Preferably, the concentration of the surfactant is from between about 1.5% (w/v) and about 10% (w/v), more preferably about 2% (w/v), and, the solubilization reagent may further comprise from between about 0% and about 25% saponin, preferably 2%.

It is to be understood that the use of reagents to treat a biological test sample prior to the use thereof in an analytical system, such as described herein, will be present in the biological test sample during subsequent analysis thereof. Accordingly, the solubilization reagent of the present invention is particularly useful where the biological test sample is to be employed in an analytical system employing specific binding proteins or antibodies which are sensitive to the presence of, for example, detergents or other pretreatment reagents which are typically employed for the purposes described herein. For example, the use of the solubilization reagent according to the present invention prior to the analysis thereof in an immunoassay system minimizes denaturation of antibody reagents employed therein, thereby having substantially no effect on the binding activity of such antibody reagents.

When employing the solubilization reagent of the present invention for performing an immunoassay, the test sample is first treated with the solubilization reagent wherein cellular populations present in the test sample are lysed and the hydrophobic analyte dissociated from other components as described above. The resulting solution is then treated with a precipitation reagent, such as described in the copending U.S. Patent Application Serial No. 567,853 , entitled "Protein Precipitation Reagent", filed on August 15, 1990 and incorporated by reference herein. Such precipitation reagent precipitates any interfering proteins, including the cellular material resulting from treatment of the test sample with the solubilization reagent of the present invention. Although the precipitated material resulting from such pretreatment step with the precipitation reagent may settle by gravity, extraction of the resulting dissociated analyte is preferably accomplished by centrifuging the treated test sample wherein the resulting supernatant contains the desired analyte, substantially free of such cellular material and components. The supernatant is then combined with a detectable tracer compound as would be known by one skilled in the art, and an appropriate antibody to, or binding agent for, the analyte prepared according to methods known in the art. According to such general immunoassay procedure, the analyte present in the test sample and the tracer compound compete for a limited number of binding sites, resulting in the formation of analyte and tracer compound complexes. By maintaining a constant concentration of the tracer compound and the antibody, the ratio of the formation of analyte complex to tracer complex is directly proportional to the amount of analyte present in the test sample.

The solubilization reagent of the present invention is particularly useful in fluorescence polarization immunoassay systems wherein the amount of analyte in a test sample is determined by exciting an assay mixture with polarized light and measuring the polarization of the fluorescence emitted by any of the free or unbound tracer compound and tracer-antibody complex. Any of the tracer compound which is not complexed to an antibody is free to rotate in less than the time required for adsorption and re-emission of fluorescent light. As a result, the re-emitted light is relatively randomly oriented so that the fluorescence polarization of any of the tracer compound not complexed to the antibody is low, approaching zero. Upon complexing with a specific antibody, the tracer-antibody complex thus formed assumes the rotation of the antibody molecule, which is slower than that of the relatively small tracer compound molecule, thereby increasing the polarization observed. When making such determination, the analyte competes with the tracer compound for antibody sites wherein the observed polarization of fluorescence of the tracer-antibody complex becomes a value between the value of the free tracer compound and the value tracer-antibody complex. Accordingly, if the test sample contains a high concentration of analyte, the observed polarization value is closer to that of the free tracer compound, i.e., low. Conversely, if the test sample contains a low concentration of analyte, the polarization value is closer to that of the tracer-antibody complex, i.e., high. By sequentially exciting the reaction mixture of an immunoassay with vertically and then horizontally polarized light, and analyzing only the vertical component of the emitted light, the polarization of the fluorescence in the reaction mixture can be accurately determined. The precise relationship between polarization and concentration of the analyte is established by measuring the polarization values of calibrators having known concentrations, and the concentration of the analyte can be interpolated from a standard curve prepared therefrom.

When employing fluorescence polarization techniques, the results can be quantified in terms of "millipolarization units", "span" (in millipolarization units) and "relative intensity". The measurement of millipolarization units indicates the maximum polarization when a maximum amount of the tracer compound is bound to the antibody in the absence of any phenylclorobenzene (PCB) in the test sample. The higher the net millipolarization units, the better the binding of the tracer compound to the antibody. For the purposes of the present invention, a net millipolarization value of at least about 130 is preferred.

The "span" is an indication of the difference between the net millipolarization and the minimum amount of the tracer compound bound to the antibody. A larger span provides for a better numerical analysis of the data. For the purposes of the present invention, a span of at least about 15 millipolarization units is preferred.

The "relative intensity " is a measure of the strength of the fluorescence signal above the backgound fluorescence. Thus, a higher intensity will give a more accurate measurement. The intensity is determined as the sum of the vertically polarized intensity plus twice the horizontally polarized intensity. The intensity can range from a signal of about three times to about thirty times the backgound noise, depending upon the concentration of the tracer compound and other assay variables. For the purpose of the present invention, an intensity of about three to about twenty times that of background noise is preferred.

The solubilization reagent according to the present invention is particularly useful for performing a fluorescent polarization immunoassay for cyclosporine and metabolites thereof employing a fluorescent tracer compound comprising 4-aminomethylfluorescein coupled to the hydroxyl group of MeBmt at the first position of cyclosporine, as described by the copending U.S. Patent Application Serial No. 567,842 , entitled "Immunoassay Reagents And Method For Determining Cyclosporine", filed on even date herewith and incorporated by reference herein, and a monoclonal antibody to cyclosporine, such as described by International Patent Application Publication No. WO 86/02080. According to such method, a precipitation reagent comprising zinc sulfate, ethylene glycol and methanol, such as described in the copending U.S. Patent Application Serial No. 567,853 , entitled "Protein Precipitation Reagent", filed on August 15, 1990 and incorporated by reference herein, a dilution buffer, and calibrators and controls are also employed. Such precipitation reagent is employed to precipitate interfering proteins, hemoglobin, and other interfering substances while, at the same time, maintaining hydrophobic analytes in solution in order to render such analytes available for binding to, for example, a specific binding protein such as an antibody.

Once the the test sample has been treated with the solubilization reagent of the present invention and the precipitation reagent as described above, the supernatant containing cyclosporine, or cyclosporine and metabolites of cyclosporine, is then combined with the antibody. Prior to addition of the tracer compound and dilution buffer, a background fluorescence reading is taken, wherein after an incubation period of from between about ten minutes and about thirty minutes, a fluorescence polarization reading is taken as described above.

The present invention will now be illustrated, but is not intended to be limited, by the following example:

Fluorescent Polarization Immunoassay For Cyclosporine

Reagents

The reagents for performing a fluorescence polarization immunoassay employing the solubilization reagent according to the present invention were prepared as follows:

a) Cyclosporine Tracer Reagent:

(i) Preparation of [O-(Chloroformyl)MeBmt][1] cyclosporine (Cyclosporine chloroformate):

Cyclosporine (24.2 mg, 0.020 mmoles) was dissolved in a 25%w/w solution of phosgene in benzene (2.0 mL) in a 10mL round bottom flask fitted with stopper and stirbar. The reaction was stirred for 5 minutes to dissolve the cyclosporine, then was allowed to stand undisturbed at room temperature for 24 hours. The reaction was concentrated in vacuo, and the product could be stored as a solid at $0^{\circ}$ C for up to six months. For subsequent reactions, a 0.02M solution in DMF was used.

(ii) Preparation of [O-(Fluorescein-4'-yl-methylaminoformyl)MeBmt][1] cyclosporine:

Cyclosporine chloroformate (0.2mL, 4 moles), as a 0.02M solution in DMF as described in step (i) above was combined with 4'-aminomethylfluorescein hydrochloride (2.0 mg, 5 moles) in a stoppered vial fitted with a stirbar. Pyridine was added until the apparent pH (by moist pH paper) was approximately 7. The reaction was stirred at room temperature for 24 hours. The solvent was removed in vacuo, and the residue was taken up in methanol and loaded onto a 1mm silica gel plate. The plate was developed with 15% methanol/methylene chloride. The product band, Rf 0.55, was eluted from the silica gel with methanol.

(iii) Preparation of Tracer Reagent:

A 60 nanomolar cyclosporine tracer reagent was prepared comprising the cyclosporine tracer compound prepared according to step (ii) above in 0.1 M sodium phosphate buffer, pH 7.5, containing 0.01 % (w/v) bovine gamma globulin, 0.1 % (w/v) soldium azide, 5.0% (w/v) ethylene glycol and 0.05% (w/v) Tween™ 20.

(b) Monoclonal Antibody Formulation:

A monoclonal antibody reagent was prepared comprising mouse (ascites) monoclonal antibody to cyclosporine (Sandoz AG, Basle, Switzerland) diluted with a citrate buffer including sodium azide.

(c) Pretreatment Reagent:

A pretreatment reagent was prepared comprising O.1 M Tris™ buffer, pH 7.5, 0.1% (w/v) sodium azide, 0.5% (w/v) copper sulfate and 10.0% (w/v) 5-sulfosalicylate.

(d) Dilution Buffer:

A dilution buffer was prepared comprising 0.1 M sodium phosphate, pH 7.5, and 0.1 % (w/v) bovine gamma globulin.

(e) Whole Blood Precipitation Reagent:

A whole blood precipitation reagent was prepared comprising 60 mM zinc sulfate, 50% (w/v) methanol and 33% (w/v) ethylene glycol.

(f) Solubilization Reagent:

A solubilization reagent was prepared comprising 2.0% (w/v) Tergitol min foam 1X ™, 2.0% (w/v) saponin and 0. 1% (w/v) sodium azide.

(g) Calibrators:

Cyclosporine monoclonal whole blood calibrators were prepared comprising cyclosporine and an artificial human whole blood matrix. The calibrators were prepared at concentrations of 0.0, 100, 250, 500, 1000, and 1500 nanograms per milliliter, with sodium azide as a preservative.

(h) Controls:

Cyclosporine monoclonal whole blood controls were prepared comprising cyclosporine and an artificial whole blood matrix. The controls were prepared at concentrations of 150, 400 and 800 nanograms per milliliter with sodium azide as a preservative.

Cyclosporine Whole Blood FPIA Assay Protocol

A fluorescent polarization immunoassay for determining cyclosporine in a whole blood test sample employing an Abbott TDx[R] Therapeutic Drug Monitoring Analyzer was performed as follows:

One hundred-fifty microliters each of patient whole blood samples containing cyclosporine, controls and calibrators were pipetted into labeled centrifuge tubes, and 50 microliters of the solubilization reagent were added to each of the tubes. A pipette was filled with the whole blood precipitation reagent, purged of air bubbles, and 300 microliters were dispensed into each centrifuge tube by touching the end of the pipette tip to the wall of each centrifuge tube while dispensing the reagent. The centrifuge tubes were then capped and mixed on a vortex mixer for ten seconds and placed into a centrifuge head so that the tubes were evenly distributed so that the centrifuge head was balanced. The tubes were centrifuged for approximately five minutes at 9,500 x g until a clear supernatant and a hard, compact pellet of denatured protein was obtained. After centrifugation was complete, each tube was uncapped and the supernatant was decanted into the corresponding sample well of a TDx Sample Cartridge.

The fluorescence polarization value of each calibrator, control and sample was determined and printed on the output tape of the Abbott TDx Analyzer. A standard curve was generated in the instrument by plotting the polarization, P, of each calibrator versus its concentration using a nonlinear regression analysis wherein, the concentration of each control and sample was read off the stored calibration curve (Figure 1) and printed on the output tape.

The sensitivity of the preferred fluorescence polarization assay according to the present invention is 15.0 nanograms/milliliter of cyclosporine and metabolites. When compared to an available radioimmunoassay using 60 clinical samples, a linear least squared regression analysis gave a slope of 0.947, an intercept of 7.15, and a correlation coefficient of 0.969.

Where a test kit according to the present invention is being used in conjunction with the TDx Analyzer, the reagents for performing the fluorescent polarization immunoassay according to the present invention can be contained in separate vials of a TDx Reagent Pack wherein vial caps from each of the vials in the Reagent Pack are removed and placed into designated wells inside the Reagent Pack. Accordingly, once the Reagent Pack is placed inside the TDx Analyzer, the assay procedure heretofore is fully automated.

If a manual assay is being performed, the test sample is first treated with the precipitation reagent as described above, and then mixed with the dilution buffer. The antibody reagent and the pretreatment solution are then placed into the test tube containing the sample, and a background fluorescence reading is taken. The tracer compound and dilution buffer are added to the sample, and after incubation, a fluorescence polarization reading is taken.

It will be apparent that many modifications and variations of the present invention as herein set forth are possible without departing from the spirit and scope hereof, and that, accordingly, such limitations are imposed only as indicated by the appended claims.

## Claims

1. A solubilization reagent useful for dissociating hydrophobic analytes from components of a biological test sample, said reagent comprising a non-ionic or a zwitterionic surfactant.

2. The reagent of claim 1 wherein said non-ionic surfactant is a non-ionic polyglycol surfactant.

3. The reagent of claim 1 wherein said zwitterionic surfactant is selected from the group consisting of N-tetradecyl-N,N-demethyl-3-ammonio-1-propanesulfonate, N-octyl-N,N-dimethyl-3-ammonio-1-propanesulfonate, N-decyl-N,N-dimethyl-3-ammonio-1-propanesulfonate, N-dodecyl-N,N-dimethyl-3-ammonio-1-propanesulfonate and N-hexadecyl-N,N-dimethyl-3-ammonio-1-propanesulfonate.

4. The reagent of claims 1-3 further comprising a glycoside.

5. The reagent of claims 1-4 comprising from between about 1.5% (w/v) and about 10% (w/v) of said surfactant in aqueous solution.

6. The reagent of claim 4 comprising less than about 25% (w/v) of said glycoside.

7. An immunoassay method for determining hydrophobic analytes in a biological test sample characterized in that said assay comprises a solubilization reagent according to claims 1-3.

8. The immunoassay method of claim 7 wherein said reagent further comprises a glycoside.

9. The immunoassay method of claim 7 wherein said reagent comprises from between about 1.5% (w/v) and about 10% (w/v) of said surfactant in aqueous solution.

10. The immunoassay method of claims 7-9 wherein said reagent comprises less than about 25% (w/v) of said glycoside.

FIG. I